Europäisches Patentamt

European Patent Office

Office européen des brevets

(19)

(11) EP 0 745 090 B1

(12) EUROPEAN PATENT SPECIFICATION

(45) Date of publication and mention
of the grant of the patent:
27.08.1997 Bulletin 1997/35

(21) Application number: 95911046.1

(22) Date of filing: 17.02.1995

(51) Int Cl.$^6$: **C07H 21/00**, A61K 31/70

(86) International application number:
PCT/US95/02080

(87) International publication number:
WO 95/22553 (24.08.1995 Gazette 1995/36)

(54) **OLIGONUCLEOTIDES WITH ANTI-RESPIRATORY SYNCYTIAL VIRUS ACTIVITY**

OLIGONUKLEOTIDE MIT WIRKUNG GEGEN DAS RESPIRATORISCHE SYNZYTIAL VIRUS

OLIGONUCLEOTIDES AYANT L'ACTIVITE CONTRE LE VIRUS RESPIRATOIRE SYNCITIAL

(84) Designated Contracting States:
AT BE CH DE DK ES FR GB GR IE IT LI LU MC NL
PT SE

(30) Priority: 18.02.1994 US 199503

(43) Date of publication of application:
04.12.1996 Bulletin 1996/49

(73) Proprietor: HYBRIDON, Inc.
Cambridge, MA 02139 (US)

(72) Inventors:
• KILKUSKIE, Robert, E.
Shrewsbury, MA 01545 (US)
• BROWN-VARGAS, Patrick, E.
Worcester, MA 01609 (US)

(74) Representative: Vossius, Volker, Dr. et al
Dr. Volker Vossius,
Patentanwaltskanzlei - Rechtsanwaltskanzlei,
Holbeinstrasse 5
81679 München (DE)

(56) References cited:
WO-A-93/07882

• VIROLOGY, vol. 185, 1991 pages 615-624, MINK
M.A. ET AL 'Nucleotide Sequences of the 3'
Leader and 5' Trailer Regions of Human
Respiratory Syncytial Virus Genomic RNA'
• VIROLOGY (1990), 174(1), 126-34 CODEN:
VIRLAX;ISSN: 0042-6822, 1990 CRISTINA, JUAN
ET AL 'Analysis of genetic variability in human
respiratory syncytial virus by the RNase A
mismatch cleavage method: subtype divergence
and heterogeneity'
• VIROLOGY (1990), 174(1), 126-34 CODEN:
VIRLAX;ISSN: 0042-6822, 1990 CRISTINA, JUAN
ET AL 'Analysis of genetic variability in human
respiratory
• syncytial virus by the RNase A mismatch
cleavage method: subtype divergence and
heterogeneity'

SEQUENCE LISTING

(1) GENERAL INFORMATION:

(i) APPLICANT: Kilkuskie, Robert E.
     Brown-Vargas, Patrick E.

(ii) TITLE OF INVENTION: Oligonucleotides Having Anti-Respiratory
Syncytial Virus
Activity

(iii) NUMBER OF SEQUENCES: 7

(iv) CORRESPONDENCE ADDRESS:

    (A) ADDRESSEE: Lappin & Kusmer
    (B) STREET: 200 State Street
    (C) CITY: Boston
    (D) STATE: Massachusetts
    (E) COUNTRY: USA
    (F) ZIP: 02109

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: PatentIn Release #1.0, Version #1.25

(vi) CURRENT APPLICATION DATA:

    (A) APPLICATION NUMBER:
    (B) FILING DATE:
    (C) CLASSIFICATION:

(viii) ATTORNEY/AGENT INFORMATION:

    (A) NAME: Kerner, Ann-Louise
    (B) REGISTRATION NUMBER: 33,523
    (C) REFERENCE/DOCKET NUMBER: HYZ-016PCT

(ix) TELECOMMUNICATION INFORMATION:

    (A) TELEPHONE: 617-330-1300
    (B) TELEFAX: 617-330-1311

(2) INFORMATION FOR SEQ ID NO:1:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:1:

```
ACGCGAAAAA ATGCGTACAA                                    20
```

(2) INFORMATION FOR SEQ ID NO:2:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:2:


TAAACCAAAA AAATGGGGCA                                                      20


(2) INFORMATION FOR SEQ ID NO:3:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:3:


AAATGGGGCA AATAAGAATT                                                      20


(2) INFORMATION FOR SEQ ID NO:4:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:4:


AAAAATGGGG CAAATAAATC                                                      20


(2) INFORMATION FOR SEQ ID NO:5:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:5:

```
CTCAATGAAT TGCTGCCCAT                                    20
```

(2) INFORMATION FOR SEQ ID NO:6:

(i) SEQUENCE CHARACTERISTICS:

    (A) LENGTH: 15 base pairs
    (B) TYPE: nucleic acid
    (C) STRANDEDNESS: single
    (D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:6:

```
TGATTTATTT GCCCC                                         15
```

(2) INFORMATION FOR SEQ ID NO:7:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:7:

```
TGAATTGATT TATTTGCCCC                                    20
```

## Description

This invention relates to respiratory syncytial virus infection. More particularly, this invention relates to oligonucleotides complementary to portions of the genome of the virus which, when hybridized to it, inhibit viral replication.

Respiratory syncytial virus (RSV is a major cause of pneumonia, bronchiolitis, and other respiratory diseases in infants, children, and even adults (Hruska et al. (1980) *Antimicrob. Ag. Chemother.* **17:**770-775; Kim et al. (1973) *Am. J. Epidem.* **98:**216-225; McIntosh et al. (1985) *Virol.* **1985:**1285-1304). Mortality resulting from RSV infection has been estimated as 0.5% in infected children (Report to the Medical Research Council (1978) *Br. Med. J.* **2:**796-798), but this may increase significantly in immunocompromised infants (Hall et al. (1981) *Pediatr.* **15:**613) or those with congenital heart disease (MacDonald et al. (1982) *N. Engl. J. Med.* **307:**397-400) .

RSV is a member of the Paramyxoviridae family and comprises the genus *Pneumovirus.* It is an enveloped virus approximately 150 to 300 nm in size, so named because virus replication leads to fusion of neighboring cells into large multinucleated syncytia (Chanock et al. (1957) *Am. J. Hyg.* **66:**281-290). The single-stranded RNA genome codes for ten virus-specific proteins. This negative stranded genome is contained in a helical nucleocapsid surrounded by a lipid envelope bearing two glycoproteins, one of which is the fusion protein which facilitates entry of virus into the cell by fusing host and viral membranes.

Upon entry, portions of the RSV genomic RNA are transcribed and translated, yielding viral proteins that mediate subsequent steps in virus reproduction. The next step is replication of the genome, which provides additional templates for transcription and further genome replication, amplifying the populations of virus-specific macromolecules within the cell, and supplying progeny genomes in large amounts for incorporation into new virus particles.

In gemome replication, the entire base sequence of the negative-strand RNA template must be conserved as a single entity. Therefore, the sequences that are not expressed as mRNA during transcriptive RNA synthesis must be incorporated into the positive-strand RNA (antigenome) that serves as an intermediate template for the synthesis of progeny negative-strand genomes. This requires that the RNA synthesizing machinery enter an antitermination mode, ignoring all of the signals at gene boundaries and an the boundary between the leader RNA template and various viral genes.

Cristina et al. showed that antigenic subtypes of RSV correlate with genetically distinct viral groups. (Cristina et al. (1990) *Virology* **174:**126-134). In this taxonomic study they used long RSV antisense probes being at least 169 nucleotides long which were synthesized in vitro by an RNA polymerase.

At present, the only clinically recognized treatment for RSV infection is the broad-spectrum antiviral drug ribavirin (1-β-D-ribofuranosyl-1,2,4-triazole-3-carb-oxamide), a synthetic nucleoside resembling guanosine. It has been proposed that this drug functions by decreasing the intracellular concentration of GTP due to competitive inhibition of IMP dehydrogenase and two virus-specific actions, by inhibiting the function of virus-coded RNA polymerases necessary to prime and elongate viral mRNAs (reviewed in Gilbert et al. (1986) *Antimicrob. Ag. Chemother.* **30:**201-205). When administered in aerosol form, ribavirin has been reported to reduce the severity of illness and the amount of virus shed (Hall et al. (1983) *N. Eng. J. Med.* **308:**1443-1447). However there is as yet no evidence that it decreases the duration of hospitalization or diminishes the need for supportive therapies.

Studies in experimental animals suggest that it may be possible to use immunotherapy for the prevention or treatment of serious RSV lower respiratory tract disease of infants and young children (Prince et al. (1985) *Virus Res.* **3:** 193-206; Prince et al. (1987) *J. Virol.* **61:**1851-1854; McIntosh et al., "Respiratory Syncytial Virus" in *Virology* (2d ed.) Raven Press, New York (1990) pp.1045-1072). However, the unusual age distribution of RSV bronchiolitis and pneumonia constitutes a major obstacle to the development of an effective vaccine for prevention of serious disease during very early infancy. If a vaccine is to have a major impact on RSV infection, it must be capable of stimulating effective resistance by the second month of life, since the peak incidence of disease occurs at this time (Parrott et al. (1973) *Am. J. Epidemiol.* **98:**289-290). Unfortunately in this age group immunosuppression by maternally derived antibodies and immunologic immaturity act to decrease vaccine efficacy (Murphy et al. (1986) *J. Clin. Microbiol.* **23:**1009-1014; Murphy (1988) *J. Virol.* **62:**3907-3910). Vaccine safety and reactogenicity, especially in the case of live vaccines, may also pose special problems in this group. Hence, alternative antiviral strategies are needed.

Recently, new chemotherapeutic agents have been developed which are capable of modulating cellular and foreign gene expression. These agents, called antisense oligonucleotides, bind to a target single-stranded nucleic acid molecules according to the Watson-Crick or the Hoogsteen rule of base pairing, and in doing so, disrupt the function of the target by one of several mechanisms: by preventing the binding of factors required for normal transcription, splicing, or translation; by triggering the enzymatic destruction of mRNA by RNase H, or by destroying the target via reactive groups attached directly to the antisense oligonucleotide.

Antisense oligodeoxynucleotides have been designed to specifically inhibit the expression of HIV-1, influenza, and other viruses (see, e.g., Agrawal et al., U.S. Patent No. 5,194,428; Pederson et al., U.S. Patent Nos. 5,149,797; Agrawal (1992) *Trends in Biotechnology* **10:**152-158; Agrawal et al. in *Gene Regulation: Biology of Antisense RNA and DNA* (Erickson and Izant, eds.) Raven Press Ltd., New York (1992) pp. 273-283); Matsukura et al . in *Prospects for Antisense*

*Nucleic Acid Therapy of Cancer and AIDS,* Wiley-Liss, Inc. (1992) pp. 159-178; and Agrawal (1991) in *Prospects for Antisense Nucleic Acid Therapy for Cancer and AIDS,* (Wickstrom, ed.) Liss, New York, pp. 145-148).

The technical problem underlying the present invention is the development of oligonucleotides that are capable of inhibiting the replication or propagation of respiratory syncytial virus and whose uses are accompanied by low or no cellular toxicity.

Thus, the present invention provides synthetic oligonucleotides which hybridize under normal physiological conditions to a portion of the negative strand of the respiratory syncytial virus genomic RNA (vRNA). As used herein, the term "synthetic oligonucleotide" denotes chemically synthesized polymers of 12 to about 60 ribonucleotide and/or deoxyribonucleotide monomers connected together or linked by at'least one 5' to 3' internucleotide linkage.

In some aspects of the invention, the oligonucleotides are modified. The term "modified oligonucleotide" is used herein as an oligonucleotide in which at least two of its nucleotides are covalently linked via a synthetic linkage, i.e., a linkage other than a phosphodiester between the 5' end of one nucleotide and the 3' end of another nucleotide in which the 5' nucleotide phosphate has been replaced with any number of chemical groups. Preferable synthetic linkages include alkylphosphonates, phosphorothioates, phosphorodithioates, alkylphosphonothioates, phosphoramidates, phosphate esters, carbamates, carbonates, phosphate triesters, acetamidate, and carboxymethyl esters. In one preferred embodiment of the invention, the oligonucleotide comprises at least one phosphorothioate linkage.

The term "modified oligonucleotide" also encompasses oligonucleotides with a modified base and/or sugar. For example, a 3', 5'-substituted oligonucleotide is a modified oligonucleotide having a sugar which, at both its 3' and 5' positions is attached to a chemical group other than a hydroxyl group (at its 3' position) and other than a phosphate group (at its 5' position). A modified oligonucleotide may also be a capped species. In addition, unoxidized or partially oxidized oligonucleotides having a substitution in one nonbridging oxygen per nucleotide in the molecule are also considered to be modified oligonucleotides. Also considered as modified oligonucleotides are oligonucleotides having nuclease resistance-conferring bulky substituents at their 3' and/or 5' end(s) and/or various other structural modifications not found *in vivo* without human intervention are also considered herein as modified.

The oligonucleotides of the invention are complementary to a portion of the vRNA and hybridize to the vRNA under normal physiological conditions existing within a cell harboring the vRNA. Such conditions include pH, temperature, and ionic conditions.

The oligonucleotides are complementary to a contiguous sequence of 15 to 25 nucleotides of the RSV vRNA. The region of the RSV genome is at least a portion of the leader region, NS1, NS2, or P RSV genes of RSV such as nucleotides (starting from the 3' end of vRNA) 1-20 (leader region), 31-50 or 41-60 (NS1/leader), and 590-609 (NS2/P). Oligonucleotides complementary to these regions have the nucleotides sequences set forth herein as SEQ ID NO:1, SEQ ID NO:2, SEQ ID NO:3, and SEQ ID NO:4, respectively.

In some aspects, the invention provides an oligonucleotide which has antiviral activity against respiratory syncytial virus effected by hybridization with a portion of the vRNA. In others, a pharmaceutical composition including at least one of the oligonucleotides of the invention, and in some embodiments, at least two different oligonucleotides of the invention, and a pharmaceutically acceptable carrier are provided. In yet other embodiments, the composition further includes ribavirin.

The pharmaceutical composition is used in a method of inhibiting, preventing, and/or reducing RSV replication in a cell. In this method, a therapeutic amount of the pharmaceutical composition is administered to the cell which is to be protected from infection or treated for an existing infection. The oligonucleotide (or oligonucleotides) in the pharmaceutical composition enters the cell, wherein it (they) hybridize(s) to RSV vRNA, thereby inhibiting RSV replication. Ribavirin in ribavirin-containing compositions inhibits replication of vRNA in the cell in three possible ways: it causes a decrease in the intracellular concentration of GTP; it inhibits 5'-cap formation of mRNAs; and/or it inhibits the function of virus-coded RNA polymerases. The pharmaceutical composition is also utilized in a method of treating respiratory syncytial virus infection wherein the composition is administered to an infected mammal or cell.

## BRIEF DESCRIPTION OF THE DRAWINGS

The foregoing and other objects of the present invention, the various features thereof, as well as the invention itself may be more fully understood from the following description, when read together with the accompanying drawings in which:

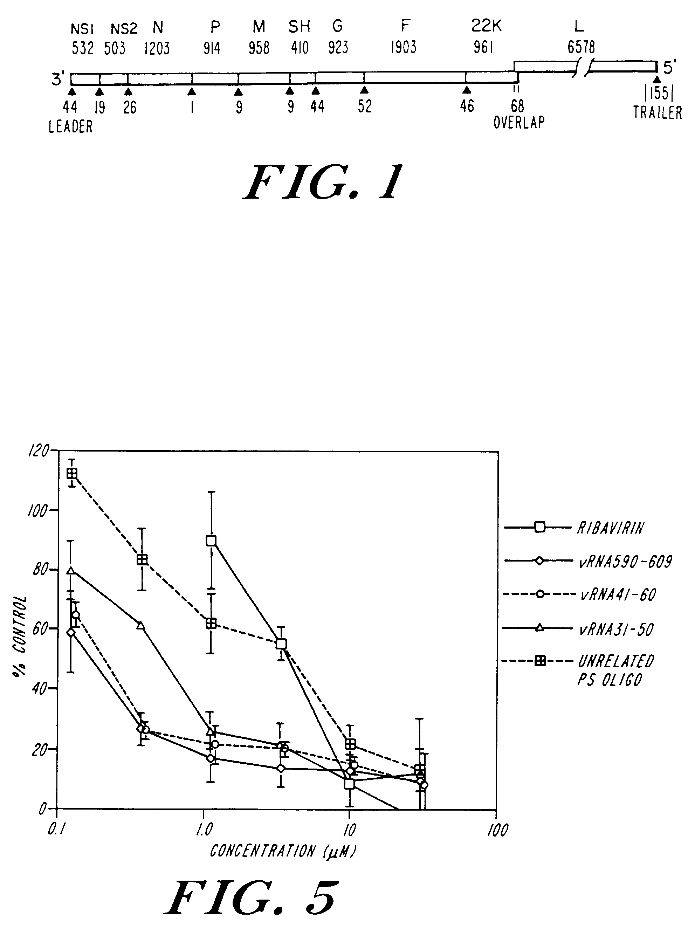

FIG. 1 is a diagrammatic representation of the genomic map of RSV (3' to 5'), wherein gene boundaries are indicated by vertical lines and gene lengths of intergenic, gene overlap, and proposed leader and trailer regions are indicated in number of bases beneath the map;

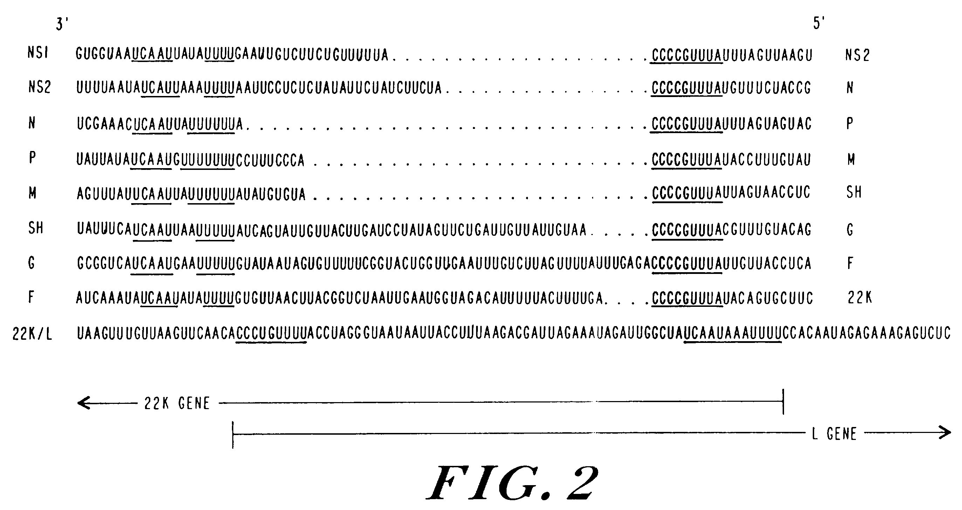

FIG. 2 is a schematic representation of the partial nucleotide sequences of the RSV genes NS1, NS2, N, P, M, SH, G, F, 22K, and L;

FIG. 3 is a graphic representation of the effect of various oligonucleotides on RSV replication *in vitro:* -□-, unrelated oligonucleotide, -◊-, vRNA 1-20 (SEQ ID NO:1); -o-, vRNA 590-609 (SEQ ID NO:4); -Δ-, vRNA 41-60 (SEQ ID NO:3);

FIG. 4 is a graphic representation of the effect of ribavirin (-□-) and various oligonucleotides: -◊-, NS2 1-20 (SEQ ID NO:6); -o-, NS2 aug (SEQ ID NO:5); -Δ-, vRNA 31-50 (SEQ ID NO:2) on RSV replication *in vitro;*

FIG. 5 is a graphic representation of the effect of ribavirin (-□-) and various oligonucleotides: -◊-, vRNA 590-609 (SEQ ID NO:41; -o-, vRNA 41-60 (SEQ ID NO:3); -Δ-, vRNA 31-50 (SEQ ID NO:2); and (-□-), unrelated oligonucleotide on RSV replication *in vitro;*

FIG. 6 is a graphic representation of the percent difference between the expected inhibition of RSV replication and the measured inhibition of RSV replication obtained when two different oligonucleotides of the invention are used in combination; and

FIG. 7 is a graphic representation of the percent difference between the expected inhibition of RSV replication and the measured inhibition of RSV replication obtained when one oligonucleotide of the invention and ribavirin are used in combination.

## DETAILED DESCRIPTION OF THE PREFERRED EMBODIMENTS

The patent and scientific literature referred to herein establishes the knowledge that is available to those with skill in the art.

The present invention relates to oligonucleotides which are sufficiently complementary to regions of the RSV genome such that, under normal physiological conditions existing in the cell, they hybridize to those regions, rendering the RSV genome unavailable to serve as a template for production of another genome strand or mRNA. As a result, RSV replication is inhibited.

The oligonucleotides are complementary to a portion of the leader region including nucleotides 1-20 (from the 3' end of the vRNA), regions of the NS1 gene, including nucleotides 31-50, and 41-60, and regions of the NS2/P genes, including nucleotides 590-609. These oligonucleotides are shown in TABLE 1 below and have the nucleotides sequences set forth herein as SEQ ID NO:1, SEQ ID NO:2, SEQ ID NO:3, and SEQ ID NO:4, respectively.

Oligonucleotides of the invention are composed of deoxyribonucleotides, ribonucleotides, or a combination of both, with the 5' end of one nucleotide and the 3' end of another nucleotide being covalently linked. These oligocnucleotides are 12 to 60 nucleotides long, with 15 to 25mers being the most common. They can be prepared by methods such as the phosphoramidate or H-phosphonate method which can be carried out manually or by an automated synthesizer as described in Uhlmann et al. (*Chem. Rev.* (1990) **90:**534-583).

The oligonucleotides of the invention may also be modified in a number of ways without compromising their ability to hybridize to RSV vRNA. For example, the oligonucleotides may contain other than phosphodiester internucleotide linkages between the 5' end of one nucleotide and the 3' end of another nucleotide in which the 5' nucleotide phosphate has been replaced with any number of chemical groups. Examples of such chemical groups include alkylphosphonates, phosphorothioates, phosphorodithioates, alkylphosphonothioates, phosphoramidates, phosphate esters, carbamates, acetamidate, carboxymethyl esters, carbonates, and phosphate triesters. Oligonucleotides with these linkages can be prepared according to known methods (see, e.g., Uhlmann et al. (1990) *Chem. Rev.* **90:**543-583).

Other modifications include those which are internal or at the end(s) of the oligonucleotide molecule and include additions to the molecule of the internucleoside phosphate linkages, such as cholesteryl or diamine compounds with varying numbers of carbon residues between the amino groups and terminal ribose, deoxyribose and phosphate modifications which cleave, or crosslink to the opposite chains or to associated enzymes or other proteins which bind to the viral genome. Examples of such modified oligonucleotides include oligonucleotides with a modified base and/or sugar such as arabinose instead of ribose, or a 3', 5'-substituted oligonucleotide having a sugar which, at both its 3' and 5' positions is attached to a chemical group other than a hydroxyl group (at its 3' position) and other than a phosphate group (at its 5' position) . Other modified oligonucleotides are capped with a nuclease resistance-conferring bulky substituent at their 3' and/or 5' end(s), or have a substitution in one nonbridging oxygen per nuclectide.

Modifications of the oligonucleotides can be an some or all of the internucleoside linkages, as well as at either or both ends of the oligonucleotide and/or in the interior of the molecule.

The preparation of these modified and unmodified oligonucleotides is well known in the art (reviewed in Agrawal et al. (1992) *Trends Biotechnol.* **10:**152-158; Agrawal in Protocols for Oligonucleotides and Analogs, Synthesis and Properties (Agrawal, ed.), Humana Press, Totowa, New Jersey (1993), Chapter 20). For example, nucleotides can be covalently linked using known methods such as the phosphoramidate, H-phosphonate, or methylphosphoramidate methods (*see, e.g.,* Uhlmann et al. (1990) *Chem. Rev.* **90:**543-584; Agrawal et al. (1987) *Tetrahedron. Lett.* **28:**(31): 3539-3542); Caruthers et al. (1987) *Meth. Enzymol.* **154:**287-313; U.S. Patent 5,149,798). Oligomeric phosphorothioate analogs can be prepared using known methods in the field such as the methoxyphosphoramidite (*see, e.g.,* Agrawal et al. (1988) *Proc. Natl. Acad. Sci.* (USA) **85:**7079-7083) or diphosphonate (*see, e.g.,* Froehler (1986) *Tetrahedron Lett.* **27:**5575-5578) method. The synthetic methods described in Bergot et al. (*J. Chromatog.* (1992) **559:**35-42) can also be used.

The present invention further provides pharmaceutical compositions having antiviral activity against RSV, and including the oligonucleotides of the present invention, along with a physiologically acceptable carrier.

As used herein, a "physiologically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the pharmaceutical compositions is contemplated. Supplementary active ingredients can also be incorporated into the compositions.

The pharmaceutical composition may be used to inhibit, reduce or prevent the replication of RSV in cells through the activity of the oligonucleotides in the composition. RSV-infected cell are then treated with the pharmaceutical composition in an amount sufficient to enable the binding of the oligonucleotide to the vRNA in the infected cells. In this way, the binding of the oligonucleotide to the RSV RNA inhibits the expression and replication of the virus. Thus the oligonucleotides according to the invention can be used to control RSV infection in infected cell cultures, thus allowing cultures to be used for other purposes.

The oligonucleotides of the invention may also be used to treat RSV infection in mammals. In this method, the pharmaceutical composition is administered once in a therapeutically effective amount or repeatedly in less than therapeutic amounts. Administration may be by intravenous or intraperitoneal injection, or by intranasal, oral, transdermal, or subcutaneous administration. Effective dosages of the oligonucleotide and modes of its administration in the treatment of RSV can be determined by routine experimentation. The pharmaceutical compositions suitable for injection or other use include sterile aqueous solutions or dispersions and sterile powders for the extemporaneous preparation of sterile injectable solutions or dispersions. In all cases the form must be sterile. It must be stable under the conditions of manufacture and storage and may be preserved against the contaminating action of microorganisms, such as bacterial and fungi. The carrier can be a solvent or dispersion medium. The prevention of the action of microorganisms can be brought about by various antibacterial and antifungal agents. Prolonged absorption of injectable pharmaceutical compositions can be brought about by the use of the compositions of agents delaying absorption.

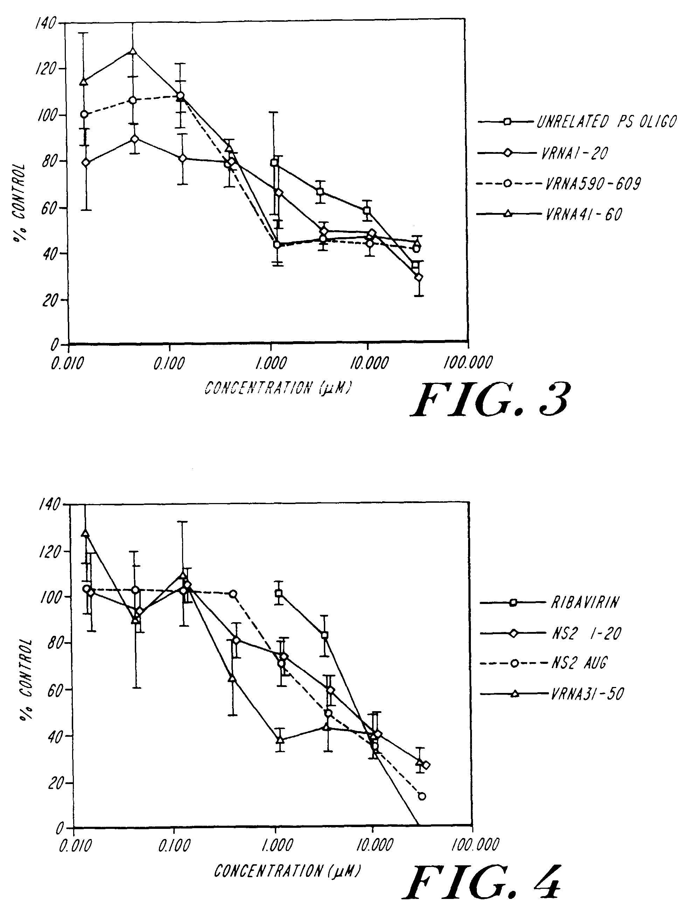

A demonstration of the antiviral effect of individual oligonucleotides of the invention is described below in the examples and is depicted in FIGS. 3 - 5. Briefly, the phosphorothioate oligonucleotides targeted to mRNA and vRNA as set forth in TABLE 1 below were synthesized and administered at several different concentrations to human epidermal carcinoma cells which had been preinfected with RSV. Virus was removed and infected cells were incubated for 3 days with either an oligonucleotide, ribavirin, or no additional treatment. The effect of the treatment was determined by plaque assay, ELISA, or virus yield assay. The ELISA results are shown in TABLE 1, where "$EC_{50}$" is the ribavirin or oligonucleotide concentration which inhibits virus replication by 50%. The oligonucleotides referred to as "NS1 aug, " "NS2 (1-15)," and "NS2 (1-20)" are complementary to mRNA transcribed from the NS1 and NS2 genes. The vRNA oligonucleotides are complementary to genomic RNA nucleotides 1-20 of the leader region and those regions encoding portions of the NS1, NS2, and P genes.

TABLE 1

| Name | SEQ ID NO. | Sequence | EC$_{50}$ Expt. # → 1 | 2 | 3 |
|---|---|---|---|---|---|
| ribavarin | -- | N/A | 10.0 | 11.0 | 7.0 |
| vRNA1-20 | 1 | ACGCGAAAAAATGCGTACAA | 16 | 5 | 3.2 |
| vRNA31-50 | 2 | TAAACCAAAAAAATGGGGCA | 1.1 | 1.1 | 0.7 |
| vRNA41-60 | 3 | AAATGGGGCAAATAAGAATT | 1.1 | 1.1 | 1 |
| vRNA590-609 | 4 | AAAAATGGGGCAAATAAATC | 1.2 | 1.1 | 1 |
| NS1 aug | 5 | CTCAATGAATTGCTGCCCAT | >30 | >30 | -- |
| NS2 (1-15) | 6 | TGATTTATTTGCCCC | 25 | 30 | -- |
| NS2 (1-20) | 7 | TGAATTGATTTATTTGCCCC | >30 | 30 | -- |

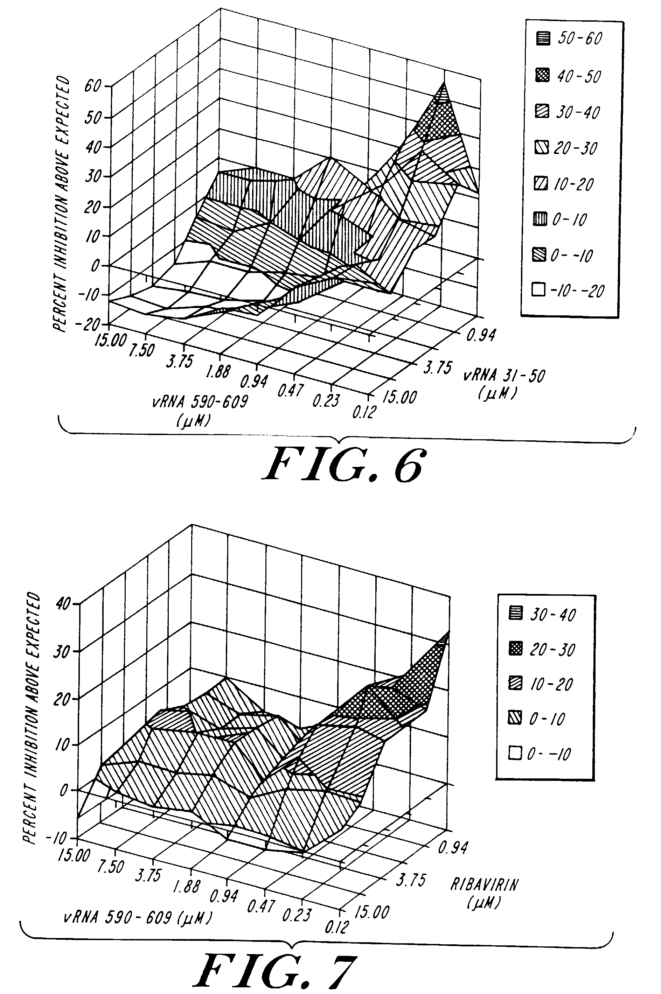

As shown in the table above, EC$_{50}$ values measured for mRNA-targeted oligonucleotides were greater than or equal to 10 μM. Antisense oligonucleotides targeted to vRNA were more potent inhibitors (EC$_{50}$ values were about 1 µM). Under the same experimental conditions, ribavirin inhibited RSV replication with an $EC_{50}$ of about 10 µM. These results indicate that the oligonucleotides of the invention targeted to RSV vRNA can effectively inhibit RSV replication. Under experimental conditions where the multiplicity of infection (MOI) was 10 fold lower, the antiviral activity of the oligonucleotides was increased (see FIG. 5).

In addition, two different oligonucleotides or one oligonucleotide and ribavirin were tested in combination. Data was analyzed according to the dissimilar site inhibitor model of Prichard et al. (*Antiviral Res.* (1990) **14:**181-206). Three dimensional graphs of percent inhibition above expected for all drug combinations were generated. The results are shown in FIGS. 6 and 7, which represent the average data from two individual experiments. These figures show that oligonucleotides of the invention can interact synergistically with ribavirin (FIG. 7) or another oligonucleotide (FIG. 6) . At low concentrations, two oligonucleotides together produce as much as 50% more inhibition than expected by adding the inhibition of the individual oligonucleotides (FIG. 6). At higher concentrations, the interactions are somewhat antagonistic. Similarly, when ribavirin was combined with an oligonucleotide, 20-30% more inhibition than expected was detected (FIG. 7). At higher concentrations, the interactions were additive. These results suggest that combinations of oligonucleotides or one oligonucleotide plus ribavirin have a therapeutic advantage over the individual compounds alone.

The following examples illustrate the preferred modes of making and practicing the present invention, but are not meant to limit the scope of the invention since alternative methods may be utilized to obtain similar results.

EXAMPLES

1. Synthesis and Purification of Oligonucleotides

Phosphodiester-linked oligonucleotides and oligonucleotide phosphorothioates were synthesized on an automated synthesizer (Gene Assembler, Pharmacia Biotech, Inc., Piscataway, NJ). They were assembled by the phosphoramidate method. The synthesis and purification of were carried out by the method of Agrawal et al. (*Proc. Natl. Acad. Sci. (USA)* (1989) **86:**7790-7794). Oligonucleotide concentrations were determined by absorbance at 260 nm, taking into account the molar extinction coefficient of the nucleotides present in each sequence (Ausubel et al., eds. (1987) *Current Protocols in Molecular Biology* (Wiley, New York)).

2. Cells

HEp-2 cells, obtained from the American Type Culture Collection (Rockville, MD, ATCC No. CLL23) were cultured in Minimum Essential Medium with Earl's balanced salts (EMEM) (JRH Biosciences, Lenexa, KA) supplemented with 10% (volume:volume) fetal bovine serum (FBS) AT 37°C in 5% $CO_2$.

3. Virus

Respiratory syncytial virus (RSV) strain A2 obtained from American Type Culture Collection, (Rockville, MD, ATCC No. VR-1302) was grown in HEp-2 cells. Virus is harvested from supernatant 4 to 6 days after infection of the cells. The supernatant was stored at -80°C and used as stock in all experiments.

4. Ribavirin

The compound was obtained from Pharmacia, Inc. (Alachua, FL).

5. Inhibition Studies with Single Compound

HEp-2 cells (20,000 cell/well of a microtiter plate) were incubated with RSV at a multiplicity of infection (moi) of 0.01 for 60 to 90 minutes at 37°C. Virus was removed and oligonucleotides (diluted in culture medium) or ribavirin are/ is added to the infected cells. Oligonucleotides were tested at 4 to 6 concentrations from 30 to 0.01 µM. Infected cells were incubated for 3 days at 37°C.

6. Inhibition Studies with Combinations of Compounds

Two oligonucleotides or one oligonucleotide and ribavirin (individually or collectively called "drugs") were evaluated in combination according to the methods of Prichard et al. (*Antiviral Res.* (1990) **14:**181-206). HEp-2 cells were infected with RSV as described above. Drugs were added to the RSV-infected HEp-2 cells in a 96 well plate. The same concentration of one drug was added to 10 wells in the top row of the plate. Serial dilutions of the drug were made into the other rows of the plate. Serial dilutions of the second drug were prepared in the columns of another 96 well plate, then transferred to the plate containing the RSV-infected cells. This established a checkerboard matrix of concentrations on the plate, containing 9 concentrations of one drug in the presence of 7 concentrations of the second drug (a total of 63 different drug combinations). In addition, the drugs were titrated alone to determine their individual effects on virus replication.

Data from drug combinations were analyzed according to the following equation for dissimilar site inhibitors as described by Prichard et al. (*ibid.*)

$$Z=X + Y(1-X)$$

This equation states that the total inhibition (Z) produced by a combination of drugs is equal to the sum of the inhibition produced by one drug (X) plus the inhibition by a second drug (Y) on the populations unaffected by the first drug (1-X).

Using this equation, the inhibition data for the individual drugs at each concentration was used to calculate expected levels of inhibition for each combination. The difference between measured inhibition and expected inhibition was determined. The percent difference between measured and expected was plotted as a function of both drugs in a 3-dimensional graph. The results are shown in FIGS. 5 and 6. Values greater than 0 suggest synergistic interaction between drugs. Values less than 0 suggest that two drugs are antagonistic. Values equal to 0 suggest that the interactions are additive.

7. Plaque Assay

Plate HEp-2 cells were seeded in 24-well plate(s) at 250,000 cells/well and incubated at 37°C overnight. Cells were washed in serum-free media. Virus was diluted in media with 2% FBS at half-log increments. Each dilution of virus (50 μl) was added to replicate wells and incubated 1-2 hours at 37°C, 5% $CO_2$. Each well was overlayed with 1 ml of 0;8% methylcellulose in media with 2% FBS and incubated at 37°C, 5% $CO_2$. When plaques developed (3-4 days), overlay was discarded and cells were stained with 5% glutaraldehyde/0.1% crystal violet (0.5 ml/well). Cells were incubated at room temperature for 45 minutes. Stain was discarded and plaques were counted under a dissecting microscope.

8. ELISA

The enzyme immunoassay procedure is based on the method of Kang and Pai (A.J.C.P. (March, 1989) 323-326) which is a modification of the methods of Berkowitz et al. (*Antimicrob. Agents Chemother.* (1985) **28:**207-210)) and Rabalais et al. (*Antimicrob. Agents Chemother.* (1987) **31:**946-948), and used monoclonal antibodies specific for RSV F glycoprotein (provided by the FDA). These RSV antibodies were diluted 1:10,000 in 1% bovine serum albumin (BSA) in phorphate buffered saline (PBS) and reacted for 1 hour at 37°C. Secondary antibody (horse radish peroxidase labelled goat anti-mouse IgG) was also diluted 1:10,000. Color was developed with 3, 3', 5, 5'-tetramethyl-benzidine (TMB) substrate, stopped with 0.1 N $H_2SO_4$, and the color read at 450 nm with a 620 nm reference filter. Virus replication as determined by this ELISA in "drug"-treated cultures was compared to untreated, infected cultures to obtain the per cent inhibition. The $EC_{50}$, or the drug concentration which inhibits viral replication by 50%, was determined graphically.

9. Virus Yield Assay

Supernatant from RSV-infected HEp-2 cells was titrated onto uninfected HEp-2 cells. The cells were then cultured for 3 to 7 days (or until syncytia were present in the culture), and the virus measured by the immunoassay described in Example 7 above. Virus titer of oligonucleotide or ribavirin-treated cells was compared to that of untreated cells.

## Claims

1. A synthetic oligonucleotide comprising from 12 to 60 covalently linked nucleotides hybridizable with a portion of the negative strand of respiratory syncytial virus (RSV) genomic RNA under physiological conditions, the portion being the viral leader region, the NS1 gene, the NS2 gene and/or the gene of RSV.

2. The oligonucleotide of claim 1, which is modified.

3. An oligonucleotide of claim 2 wherein the base and/or sugar moiety is modified.

4. The oligonucleotide of any of Claims 1 to 3, comprising at least one internucleotide linkage selected from the group consisting of alkylphosphonate, phosphorothioate, phosphorodithioate, alkylphosphonothioate, phosphoramidate, phosphate ester, carbamate, carbonate, phosphate triester, acetamidate, carboxymethyl ester, and combinations thereof.

5. The oligonucleotide of claims 1 to 4 which comprises at least one deoxyribonucleotide.

6. The oligonucleotide of claims 1 to 4 which comprises at least one ribonucleotide.

7. The oligonucleotide of claim 4 which comprises at least one phosphorothioate internucleotide linkage.

8. The oligonucleotide of claim 1 having a nucleotide sequence selected from the group consisting of SEQ ID NO:1, SEQ ID NO:2, SEQ ID NO:3, and SEQ ID NO:4.

9. A pharmaceutical composition comprising an oligonucleotide of any one of claims 1 to 8 and a pharmaceutically acceptable carrier.

10. A pharmaceutical composition comprising at least two different oligonucleotides of any one of claims 1 to 8 and a pharmaceutically acceptable carrier.

11. The pharmaceutical composition of claim 9 or 10 further comprising ribavirin.

12. A method of preventing or reducing RSV replication in a cell in vitro comprising the step of administering to the cell an inhibiting amount of the pharmaceutical composition of claim 9, 10, 11.

13. The use of at least one oligonucleotide according to any one of claims 1 to 8 for the preparation of a pharmaceutical composition.

14. The use of at least one oligonucleotide according to any one of claims 1 to 8 and ribavirin for the preparation of a pharmaceutical composition.


## Patentansprüche

1. Ein synthetisches Oligonucleotid, umfassend 12 bis 60 kovalent gebundene Nucleotide, hybridisierbar mit einem Teil des negativen Stranges der genomischen RNA des Respiratory-Syncytial-Virus (RSV) unter physiologischen Bedingungen, wobei der Teil die Leader-Region, das NS1-Gen, das NS2-Gen und/oder das P-Gen von RSV ist.

2. Oligonucleotid gemäß Anspruch 1, das modifiziert ist.

3. Oligonucleotid gemäß Anspruch 2, wobei die Base und/oder der Zuckerrest modifiziert ist.

4. Oligonucleotid gemäß einem der Ansprüche 1 bis 3, umfassend mindestens eine Internucleotidbindung, ausgewählt aus der Gruppe bestehend aus Alkylphosphonat, Phosphorothioat, Phosphorodithioat, Alkylphosphonothioat, Phosphoramidat, Phosphatester, Carbamat, Carbonat, Phosphattriester, Acetamidat, Carboxymethylester und Kombinationen hiervon.

5. Oligonucleotid gemäß Anspruch 1 bis 4, das mindestens ein Desoxyribonucleotid umfaßt.

**6.** Oligonucleotid gemäß Ansprüchen 1 bis 4, die mindestens ein Ribonucleotid umfassen.

**7.** Oligonucleotid gemäß Anspruch 4, das mindestens eine Phosphorothioat-Internucleotidbindung umfaßt.

**8.** Oligonucleotid gemäß Anspruch 1 mit einer Nucleotidsequenz, ausgewählt aus der Gruppe bestehend aus SEQ ID NO:1, SEQ ID NO:2, SEQ ID NO:3 und SEQ ID NO:4.

**9.** Ein Arzneimittel, umfassend ein Oligonucleotid gemäß einem der Ansprüche 1 bis 8 und einen pharmazeutisch-verträglichen Träger.

**10.** Ein Arzneimittel, umfassend mindestens zwei verschiedene Oligonucleotide gemäß einem der Ansprüche 1 bis 8 und einen pharmazeutisch-verträglichen Träger.

**11.** Ein Arzneimittel gemäß Anspruch 9 oder 10, ferner umfassend Ribavirin.

**12.** Ein Verfahren zur Verhinderung oder Reduzierung der RSV-Replikation in einer Zelle *in vitro,* umfassend die Schritte der Verabreichung einer inhibierenden Menge des Arzneimittels gemäß Anspruch 9, 10 oder 11 zu einer Zelle.

**13.** Die Verwendung von mindestens einem Oligonucleotid gemäß einem der Ansprüche 1 bis 8 zur Herstellung eines Arzneimittels.

**14.** Die Verwendung von mindestens einem Oligonucleotid gemäß einem der Ansprüche 1 bis 8 und Ribavirin zur Herstellung eines Arzneimittels.

## Revendications

**1.** Oligonucléotide synthétique comprenant de 12 à 60 nucléotides liés par covalence pouvant s'hybrider avec une partie du brin négatif de l'ARN du génome du virus respiratoire syncytial (RSV) dans des conditions physiologiques, la partie consistant en la région leader virale, le gène NS1, le gène NS2 et/ou le gène du RSV.

**2.** Oligonucléotide selon la revendication 1, qui est modifié.

**3.** Oligonucléotide selon la revendication 2 dans lequel le résidu base et/ou sucre est modifié.

**4.** Oligonucléotide selon l'une quelconque des revendications 1 à 3 comprenant au moins une liaison internucléotidique choisie dans le groupe constitué des liaisons alkylphosphonate, phosphorothioate, phosphorodithioate, alkylphosphonothioate, phosphoramidate, phosphoester, carbamate, carbonate, phosphotriester, acétamidate, ester de carboxyméthyle, et leurs combinaisons.

**5.** Oligonucléotide selon les revendications 1 à 4 qui comprend au moins un désoxyribonucléotide.

**6.** Oligonucléotide selon les revendications 1 à 4 qui comprend au moins un ribonucléotide.

**7.** Oligonucléotide selon la revendication 4 qui comprend au moins une liaison internucléotidique phosphorothioate.

**8.** Oligonucléotide selon la revendication 1 possédant une séquence nucléotidique choisie dans le groupe constitué des séquences SEQ ID NO:1, SEQ ID NO:2, SEQ ID NO:3 et SEQ ID NO:4.

**9.** Composition pharmaceutique comprenant un oligonucléotide selon l'une quelconque des revendications 1 à 8 et un support pharmaceutiquement acceptable.

**10.** Composition pharmaceutique comprenant au moins deux oligonucléotides différents, selon l'une quelconque des revendications 1 à 8 et un support pharmaceutiquement acceptable.

**11.** Composition pharmaceutique selon la revendication 9 ou 10 comprenant en outre de la ribavirine.

**12.** Procédé pour empêcher ou réduire la réplication du RSV dans une cellule, in vitro, comprenant l'étape consistant à administrer à la cellule une quantité inhibitrice de la composition pharmaceutique de la revendication 9, 10 ou 11.

13. Utilisation d'au moins un oligonucléotide selon l'une quelconque des revendications 1 à 8 pour la préparation d'une composition pharmaceutique.

14. Utilisation d'au moins un oligonucléotide selon l'une quelconque des revendications 1 à 8 et de ribavirine pour la préparation d'une composition pharmaceutique.

*FIG. 1*

*FIG. 5*

EP 0 745 090 B1

3'                                  5'

NSI    GUGGUAAUCAAUUAUAUUUUGAAUUGUCUUCUGUUUUUA . . . . . . . . . . . . . . . . . . . . . CCCCGUUUAUUUAGUUAAGU   NS2

NS2    UUUUAAUAUCAUUAAAUUUUAAUUCCUCUCUAUAUUCUAUCUUCUA . . . . . . . . . . . . . . CCCCGUUUAUGUUUCUACCG   N

N    UCGAAACUCAAUUAUUUUUUA . . . . . . . . . . . . . . . . . . . . . . . . . . . . CCCCGUUUAUUUAGUAGUAC   P

P    UAUUAUAUCAAUGUUUUUUUUCCUUUCCCA . . . . . . . . . . . . . . . . . . . . . CCCCGUUUAUACCUUUGUAU   M

M    AGUUUAUUCAAUUAUUUUUUUAUAUGUGUA . . . . . . . . . . . . . . . . . . . . . . . CCCCGUUUAUUAGUAACCUC   SH

SH    UAUUUCAUGAAUUAAUUUUUAUCAGUAUUGUUACUUGAUCCUAUAGUUCUGAUUGUUAUUGUAA . . . . . CCCCGUUUACGUUUGUACAG   G

G    GCGGUCAUCAAUGAAUUUUUGUAUAAUAGUGUUUUUCGGUACUGGUUGAAUUUGUCUUAGUUUUUAUUUGAGACCCCGUUUAUUGUUACCUCA   F

F    AUCAAAUAUCAAUAUAUUUUGUGUUAACUUACGGUCUAAUUGAAUGGUAGACAUUUUUACUUUUGA . . . . CCCCGUUUAUACAGUGCUUC   22K

22K/L    UAAGUUUGUUAAGUUCAACACCCUGUUUUACCUAGGGUAAUAAUUACCUUUAAGACGAUUAGAAAUAGAUUGGCUAUCAAUAAAUUUUCCACAAUAGAGAAAGAGUCUC

← —— 22K GENE ———————————————————————————————————————————————————|

|————————————————————————————————————————————————— L GENE ———→

# FIG. 2

**FIG. 3**

Legend:
- □ — UNRELATED PS OLIGO
- ◇ — VRNA1-20
- ○ — VRNA590-609
- △ — VRNA41-60

**FIG. 4**

Legend:
- □ — RIBAVIRIN
- ◇ — NS2 1-20
- ○ — NS2 AUG
- △ — VRNA31-50

17

**FIG. 6**

**FIG. 7**